# FASCICULE DE BREVET EUROPEEN

(11) **EP 0 943 010 B1**
(45) Date de publication et mention de la délivrance du brevet: **13.03.2002**
(21) Numéro de dépôt: 97912259.5
(22) Date de dépôt: 27.10.1997
(51) Int. Cl.: C12Q 1/68

(54) **PROCEDE DE MISE EN EVIDENCE DE CONTAMINANTS MICROBIOLOGIQUES VIVANTS DANS UN ECHANTILLON DE PRODUIT A USAGE ALIMENTAIRE**
VERFAHREN ZUR ERKENNUNG VON LEBENDEN MIKROBIOLOGISCHEN VERUNREINIGUNGEN IN EINER LEBENSMITTELPROBE
METHOD FOR DETECTING LIVE MICROBIOLOGICAL CONTAMINANTS IN A FOOD PRODUCT SAMPLE

(30) Priorité: 28.10.1996 FR 9613125
(43) Date de publication de la demande: 22.09.1999
(73) Titulaire: COMPAGNIE GERVAIS DANONE, 92300 Levallois Perret (FR)
(72) Inventeur: GENDRE, François, F-67200 Strasbourg (FR); BRIGNON, Pierre, F-67200 Strasbourg (FR)
(74) Mandataire: Le Forestier, Eric
(86) Numéro de dépôt international: FR9701918
(87) Numéro de publication internationale: WO9818958

(56) Documents cités:
- EP-A- 0 133 671
- WO-A-90/11370
- WO-A-92/03455
- BERG S. ET AL.,: "Development of an amplification and hybridization assay for the specific and snsitive detection of Mycoplasma fermentas DNA" MOL. CELL. PROBES, vol. 10, - février 1996 pages 7-14, XP002034547
- MULLIS K.B. ET AL.,: "PCR; The polymerase chain reaction" 1994 , BIRKHAUSER , BOSTON, USA XP002034388 voir page 277 - page 294
- DATABASE WPI Section Ch, Week 9748 Derwent Publications Ltd., London, GB; Class B04, AN 97-520740 XP002054735 & JP 09 248 186 A (OZEKI KK) , 22 septembre 1997

### LISTE DE SEQUENCES

(1) INFORMATIONS GENERALES:
   (i) DEPOSANT:
      (A) NOM: COMPAGNIE GERVAIS DANONE
      (B) RUE: 126/130 RUE JULES GUESDE
      (C) VILLE: LEVALLOIS PERRET
      (E) PAYS: FRANCE
      (F) CODE POSTAL: 92300
   (ii) TITRE DE L' INVENTION: PROCEDE DE MISE EN EVIDENCE DE CONTAMINANTS MICROBIOLOGIQUES VIVANTS DANS UN ECHANTILLON DE PRODUIT A USAGE ALIMENTAIRE
   (iii) NOMBRE DE SEQUENCES: 6
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 1:
   CGCTGGAAGG CGACGMRRAG 20
(2) INFORMATIONS POUR LA SEQ ID NO: 2:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 2:
   CGGAAGTAGA ACTGCGGACG GTAG 24
(2) INFORMATIONS POUR LA SEQ ID NO: 3:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 3:
   TCCATGGTAC AAGGGTTGGG AA 22
(2) INFORMATIONS POUR LA SEQ ID NO: 4:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 4:
   GCGAATCTAC CTAATGGTGG GT 22
(2) INFORMATIONS POUR LA SEQ ID NO: 5:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 5:
   GCTGGTATCT CCAAGGATGG 20
(2) INFORMATIONS POUR LA SEQ ID NO: 6:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 6:
   CGACGGACTT GACTTCRGTG G 21

## Description

La présente invention concerne un procédé de mise en évidence de contaminants microbiologiques vivants dans un échantillon de produits à usage alimentaire.

Les produits à usage alimentaire sont particulièrement sensibles à la contamination par des produits microbiologiques, en particulier par les bactéries, en raison de la forte incidence d'une contamination sur la santé des personnes ingérant la nourriture. Dans ce domaine, les microorganismes vivants, et donc actifs, sont particulièrement redoutables puisqu'ils peuvent ensuite se propager dans l'organisme et transmettre des maladies sévères. Il existe donc un besoin certain de mise au point d'un procédé permettant leur détection dans les produits à usage alimentaire, à la fois de façon précise et satisfaisante.

Il existe de nombreuses méthodes de détection, en particulier des bactéries, dans des échantillons. On peut citer par exemple, la mise en culture de l'échantillon afin d'augmenter le nombre de cellules bactériennes présentes jusqu'à observer des colonnies qui peuvent être comptées. Le cas échéant, la culture peut être suivie d'une étape supplémentaire permettant d'identifier le type particulier de bactéries contenues dans l'échantillon. Ces méthodes bactériologiques demandent beaucoup de temps et de qualification de la part des personnes qui les mettent en oeuvre. Ainsi par exemple, il est généralement nécessaire de procéder à une incubation de 24 à 48 heures avant d'obtenir de façon certaine un résultat positif ou négatif.

D'autres méthodes ont également été envisagées afin d'éliminer les inconvénients de la méthode bactériologique conventionnelle. Ainsi, la microscopie est fréquemment utilisée pour détecter les bactéries dans des échantillons cliniques. Habituellement il est nécessaire de colorer l'échantillon pour augmenter les limites de détection, ce qui d'une part, représente une méthode laborieuse et d'autre part, est inadapté pour des échantillons à usage alimentaire (1). Certaines méthodes immunologiques ont également été développées avec succès pour la détection de certaines espèces ayant des antigènes de surface qui peuvent être reconnus par des anticorps spécifiques. Toutefois, une telle approche ne peut être utilisée pour la détermination qualitative de bactéries dans un échantillon qui peut contenir une grande variété d'espèces de bactéries qui n'ont pas un antigène de surface commun.

La publication européenne de brevet EP-A-0 133671 décrit une méthode de détermination de la présence de bactéries dans des échantillons, en particulier dans des milieux appropriés pour faire du diagnostic, tels que des fluides corporels, qui met en oeuvre les techniques d'hybridation d'acide nucléique. Selon cette méthode, l'échantillon à tester est tout d'abord soumis à des conditions de dénaturation de façon à rendre simple brin les acides nucléiques des bactéries présentes dans l'échantillon. Les acides nucléiques mono brin ainsi obtenus sont mis en contact avec une sonde polynucléotide ayant une séquence homologue avec au moins une séquence commune à toutes les espèces de bactéries présentes dans l'échantillon. La mise en contact de la sonde et des acides nucléiques dénaturés est effectuée de façon à réaliser une hybridation entre la sonde et les séquences respectives des bactéries. Plus particulièrement, la sonde utilisée comprend au moins une portion de l'un des brins d'un gène codant pour la synthèse d'un acide nucléique ou d'une protéine qui intervient dans le mécanisme de la synthèse des protéines. Parmi ce type de gènes, sont cités en particulier des gènes codant pour des ARN de transfert, des ARN ribosomaux, des facteurs d'initiation, d'élongation ou de terminaison de la traduction. Une des caractéristiques de cette méthode, est qu'elle est basée non pas sur l'expression, mais plutôt sur la présence d'acides nucléiques hybridables, par exemple l'ARN ou l'ADN génomique ou les acides nucléiques extra chromosomaux des bactéries présentes. I1 en résulte, ce qui aux yeux du déposant constitue un avantage, que les échantillons n'ont pas à être traités de façon à garantir la viabilité des bactéries présentes.

Le document WO 92/03455 décrit des compositions et des procédés pour le traitement et le diagnostic d'infections par Candida, en particulier des nucléotides susceptibles de s'hybrider de façon spécifique avec une partie de l'ARMm de Candida, notamment l'ARNm codant pour les facteurs d'élongation 1 et 2 (TEF1 et TEF2). Ce document vise uniquement les applications thérapeutiques ou les diagnostics, soit pour détecter la présence de Candida chez un patient, soit pour inhiber l'activité de cette bactérie en bloquant l'expression de protéines essentielles.

Le document Berg et al., "MOL. CELL. PROBES, vol. 10 February 1996 pp 7-14" décrit un système de détection spécifique de l'ADN de microplasmes en utilisant la technique PCR. Bien qu'il soit indiqué, page 12 de ce document, que la séquence cible pour l'amplification PCR est le gène tuf qui code pour le facteur d'élongation Tu, d'une part ce document vise uniquement la détection d'ADN et non pas celle de l'ARNm, et d'autre part il s'agit uniquement d'une méthode de détection de bactéries pour établir un diagnostic chez un patient. Ce document n'envisage aucune application dans le domaine de l'invention où se pose le problème spécifique de la détection de bactéries vivantes.

Ainsi, il n'existe aucune méthode connue permettant la mise en évidence de contaminants microbiologiques vivants dans un produit à usage alimentaire, qui à la fois discrimine entre les microorganismes vivants et les microorganismes morts, et d'autre part ne pose pas de problèmes relatifs à la santé publique.

Les inventeurs ont maintenant découvert qu'il était possible de mettre en évidence des contaminants microbiologiques vivants dans un échantillon de produits à usage alimentaire en détectant dans cet échantillon la présence d'ARN messager (ARNm) codant pour la synthèse d'une protéine intervenant dans le mécanisme de synthèse des protéines par lesdits contaminants.

Différentes familles de microorganismes peuvent être détectées selon l'invention. De façon non limitative, on peut citer les procaryotes, en particulier les bactéries, les eucaryotes unicellulaires, en particulier les levures, les eucaryotes pluricellulaires, en particulier les champignons. Au sein de ces familles, des espèces différentes peuvent être identifiées. ainsi, par exemple, Escherichia, Salmonella, Mycobacterium pour les bactéries ; Saccharomyces, Candida pour les levures ; Mucor, Neurospora, Trichoderma pour les champignons.

La synthèse des protéines par les microorganismes comprend des étapes de transcription et de traduction. Dans le cadre de la traduction, il existe des acides nucléiques et des protéines impliqués dans chacune des trois étapes fondamentales de la synthèse des protéines, initiation, élongation et terminaison. On peut ainsi citer comme élément intervenant dans cette phase de traduction des ARN de transfert, des ARN ribosomaux, des protéines ribosomales, des facteurs d'initiation, d'élongation et de terminaison. En fait, il existe des protéines ou acides nucléiques dont la structure primaire est au moins partiellement conservée entre différentes espèces de microorganismes appartenant à une même famille et cette propriété est avantageusement mise à profit selon l'invention pour mettre en évidence des contaminants microbiologiques vivants appartenant à des espèces différentes.

Ainsi, selon un mode de réalisation préféré, l'invention a pour objet un procédé pour la mise en évidence de façon non spécifique des contaminants vivants appartenant à des espèces différentes d'une famille de microorganismes, selon lequel l'ARNm détecté est un ARNm qui code pour la synthèse d'une protéine dont la structure primaire est au moins partiellement conservée entre des espèces différentes.

Si l'on souhaite au contraire une plus grande spécificité de détection entre espèces différentes de microorganismes, il est possible selon l'invention de procéder à la détection de différents ARNm codant respectivement pour la synthèse d'une protéine dont la structure primaire n'est pas conservée entre des espèces différentes.

Parmi tous les facteurs qui interviennent dans la synthèse des protéines, un exemple particulièrement préféré est représenté par les facteurs d'élongation. Parmi ces facteurs, on peut citer les facteurs EF-1, EF-2, EF-G, EF-TU pour les bactéries (2), ou encore le facteur EF-1α (3) pour les levures et les champignons. Ces facteurs jouent un rôle fondamental dans la synthèse des protéines, en ce qu'ils permettent de déterminer la durée pendant laquelle un amino-acyl ARNt reste associé au ribosome et à la chaîne polypeptidique en formation, fonction désignée par l'expression "vérification de cinétique" ("kinetic proofreading") (4).

Il est particulièrement avantageux de rechercher la présence de l'ARN messager codant pour un facteur d'élongation pour différentes raisons. Tout d'abord, ce gène représente un marqueur très approprié de la viabilité des cellules puisque l'inactivation de ce gène est un événement léthal, à la fois chez les procaryotes et les eucaryotes (5, 6). D'autre part, le gène codant pour un facteur d'élongation code pour une protéine qui figure parmi les protéines les plus largement exprimées chez les procaryotes et les eucaryotes (7, 8) ce qui permet de diminuer considérablement le niveau de détection des cellules. Enfin, comme indiqué ci-dessus, dans la mesure où cette fonction est conservée chez les procaryotes et chez les eucaryotes et où la structure primaire de ce type de gènes est très similaire (9), il est possible de moduler la spécificité de la détection. Il est ainsi possible de mettre en oeuvre des moyens de détection permettant de distinguer entre les procaryotes et les eucaryotes ou au contraire de mettre en évidence de façon non spécifique à la fois des bactéries, des levures et/ou des champignons.

Par ailleurs, l'ARNm codant pour des facteurs d'élongation a une demi-vie très courte (10, 11). Sa présence permet donc de révéler la présence de cellules qui étaient encore vivantes environ dix minutes avant la détection de l'ARNm.

Selon un mode de réalisation préféré, on détecte donc un ARNm qui code pour un facteur d'élongation. Dans ce cas, une cellule de microorganisme vivante au sens de l'invention, est une cellule capable de produire l'ARNm corespondant à un facteur d'élongation.

Une méthode de choix pour la détection de la présence d'ARN messager selon l'invention, est la RT-PCR (polymérisation par réaction en chaîne combinée à une transcription inverse). Cette technique consiste à effectuer une PCR à partir d'un ARN préalablement transcrit en ADN complémentaire, en présence d'une transcriptase inverse et d'une amorce. Après la phase RT, la phase PCR proprement dite est effectuée dans les conditions classiques en présence de l'ADN à amplifier, des deux amorces oligonucléotidiques encadrant la région à amplifier et des quatre désoxynucléotides triphosphates (DATP, DCTP, DGTP, DTTP) en large excès molaire et de l'enzyme Taq polymérase.

Bien entendu, le choix des amorces est essentiel, puisqu'il permet de cibler l'ARNm que l'on souhaite détecter. Les amorces oligonucléotidiques sont préparées de façon telle qu'elles sont spécifiques de la région codante d'un gène codant pour un facteur d'élongation. Parmi les facteurs d'élongation connus, on choisit de préférence le facteur EF-TU pour les bactéries et le facteur EF-1α pour les levures et les champignons. C'est ainsi que l'on a par exemple préparé les amorces B1/B2 (5' CGCTGGAAGGCGACGMRRAG 3'/ 5' CGGAAGTAGAACTGCGGACGGTAG 3') qui sont spécifiques d'un fragment du facteur d'élongation EF-TU des bactéries qui se retrouve notamment chez Salmonella typhimurium, Mycobacterium tuberculosis, Mycobacterium leprae, Escherichia coli, Brevibacterium linens, Streptomyces ramocissimus.

En ce qui concerne les levures, on a préparé des amorces L1/L2 (5' TCCATGGTACAAGGGTTGGGAA 3' / 5' GCGAATCTACCTAATGGTGGGT 3') qui sont spécifiques d'un fragment du facteur d'élongation EF-1α des levures, qui se retrouve à la fois chez Saccharomyces cerevisiae et Candida albicans.

Enfin, un exemple d'amorce nucléotidique utilisable pour 1a détection d'ARN messager spécifique des champignons, est représenté par le couple M1/M2 (5' GCTGGTATCTCCAAGGATGG 3' / 5'-CGACGGACTTGACTTCRGTGG 3'). Ces amorces sont plus particulièrement spécifiques d'un fragment du facteur d'élongation EF-1α des champignons qui se retrouve chez Mucor racemosus, Neurospora crassa, Trichoderma reesei, Absidia glauca, Aureobasidium pullulans, Histoplasma capsulatum, Puccinia graminis.

Les amorces utilisables selon l'invention ont été préparées après comparaison de la région codante du facteur d'élongation de différentes espèces de microorganismes (software Lasergene, Dnastar, Medison, Wisconsin USA).

D'une manière générale, le procédé selon l'invention peut être caractérisé par l'ensemble des étapes suivantes :
a) on prélève un échantillon de produit alimentaire à tester ;
b) on lyse les cellules ;
c) on effectue une transcription inverse ;
d) on effectue des cycles d'amplification à l'aide d'amorces oligonucléotidiques specifiques de la région codante d'un gène codant pour un facteur d'élongation ;
e) on sépare les produits d'amplification ;
f) on visualise les produits d'amplification.
g) on compare les produits tels que visualisés à l'étape f) avec les produits d'amplification obtenus à partir d'ARNm pur.

Puisque l'invention vise essentiellement à détecter des contaminants microbiologiques vivants, il est particulièrement avantageux d'éliminer autant que possible, toute contamination supplémentaire du milieu par l'ADN présent dans l'échantillon. C'est pourquoi, selon un mode préféré de réalisation de l'invention, on ajoute après l'étape b) une étape supplémentaire (b') destinée à éliminer l'ADN présent dans l'échantillon. Pour cela, on peut simplement ajouter dans le milieu réactionnel de l'ADNase I qui ne contient pas de RNase. Si on souhaite vérifier l'absence de faux positifs qui seraient liés à la présence d'ADN, une possibilité consiste à réaliser une réaction PCR à partir des mêmes échantillons que ceux utilisés pour la RT-PCR.

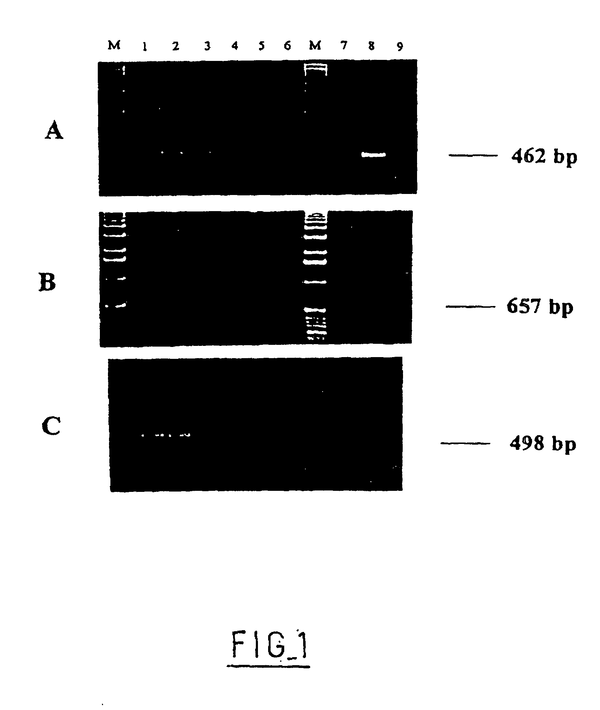

L'invention sera plus particulièrement illustrée par les modes de réalisation qui vont suivre accompagnés des figures 1 à 3.

La figure 1 représente un gel d'électrophorèse des produits de RT-PCR du gène EF-Tu de E. coli (gène tuf b) (A) ou du gène EF-1α du S. cerevisiae (B) ou de M. racemosus (C) mis en suspension dans du lait dans des concentrations allant de 105 cellules/ml (bande 1) jusqu'à 1 cellule/ml (bande 6) en diminuant à chaque fois, de 10 fois la concentration. La bande 7 représente un contrôle négatif sans cellule ; la bande 8 représente un contrôle positif avec de l'ARNm pur ; la bande 9 représente une PCR effectuée sur un lysat digéré avec l'ADNase I; les bandes M représentent une échelle de 1kb (Gibco BRLTM).

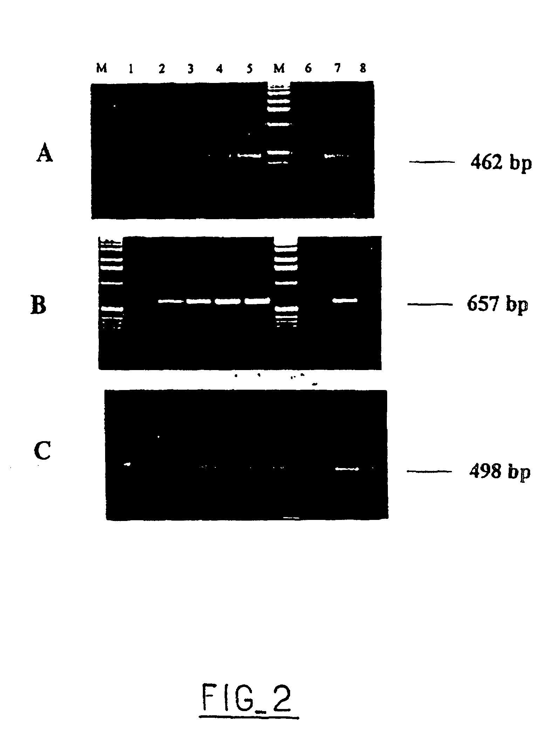

La figure 2 représente un gel d'électrophorèse des produits RT-PCR du gène EF-Tu de E. coli (gène tuf b) (A) ou du gène EF-1α de S. cerevisiae (B) ou de M. racemosus (C) mis en suspension dans du lait à une concentration cellulaire de 104-105 cellules/ml. La bande 1 correspond à 100 % de cellules mortes ; la bande 2, à 25 % de cellules vivantes et 75 % de cellules mortes ; la bande 3, à 50 % de cellules mortes et 50 % de cellules vivantes ; la bande-4, à 75 % de cellules vivantes et 25 % de cellules mortes ; la bande 5, à 100 % de cellules vivantes ; la bande 6, à un contrôle négatif sans cellule ; la bande 7, à un contrôle positif avec de l'ARNm pur ; la bande 8, à une PCR effectuée sur un lysat digéré avec l'ADNase I ; les bandes M, à une échelle de 1 kb (Gibco BRLTM).

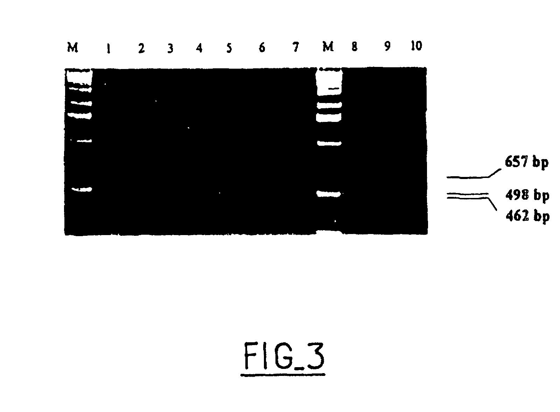

La figure 3 représente un gel d'électrophorèse des produits de RT-PCR du gène EF-Tu de E. coli (gène tuf b) et du gène EF-1α de S. cerevisiae et M. racemosus. Il est obtenu en une seule étape de RT-PCR. Les cellules ont été mises en suspension dans du lait à une concentration de 10 cellules/ml. Bande 1, amorces de bactéries ; bande 2, amorces de levures ; bande 3, amorces de champignons ; bande 4, amorces de bactéries et de levures ; bande 5, amorces de levures et de champignons ; bande 6, amorces de bactéries et de champignons ; bande 7, amorces de bactéries, levures et champignons ; bande 8, contrôle négatif sans cellule ; bande 9, contrôle positif avec de l'ARNm pur ; bande 10, PCR effectuée sur un lysat digéré avec l'ADNase I ; bandes M, échelle de 1kb (Gibco BRLTM).

Des exemples ont été réalisés avec les souches suivantes :
- Escherichia coli (souche ATCC 19110) ;
- Saccharomyces cerevisiae S288C ;
- Mucor racemosus (souche CBS 11308).

L'échantillon sur lequel le procédé a été mis en oeuvre est le lait mais bien entendu, l'invention est applicable à tout autre produit à usage alimentaire, de préférence liquide.

### EXEMPLE 1 : détermination de la présence de cellules vivantes de E. coli dans du lait (détection d'ARNm codant pour le facteur d'élongation EF-Tu)

### a) Préparation de la souche

On cultive une souche E.coli à 37°C dans un bouillon Luria, puis on prépare un milieu solide par addition d'agar à 2% (Laboratoires Difco). Une partie des cellules est prélevée pendant la phase exponentielle de croissance. Les cellules sont réparties dans des échantillons de 2ml puis sont soumises à des conditions léthales par incubation à 65°C pendant 15mn. Des dilutions en série des échantillons sont mises sur plaques sur milieu Agar afin de vérifier là viabilité des cellules et de les compter. Ensuite, les échantillons sont utilisés pour inoculer des échantillons de lait de vache pasteurisé à ultra haute température disponible commercialement.

### b) contamination du lait

On réalise des séries de concentration allant de 105 à une cellule par ml de lait. L'interférence possible du lait avec la réaction de PCR est éliminée en effectuant 4 cycles de lavage avec du milieu PBS, comme cela est indiqué dans le document Cooray et al. (12), dont le contenu est ici incorporé par référence. Le culot final de lait est resuspendu après lavage avec du PBS jusqu'à retrouver le volume original de lait puis filtré à travers une membrane de polycarbonate de 25mm de diamètre humidifiée avec du milieu PBS (taille de pore, 0.8µm Millipore). Le filtre est transféré dans un tube FalconTM de 15 ml (Becton Dickinson). On ajoute 1 ml de PBS et on mélange vigoureusement pendant 30 secondes et on récupère le surnageant. On ajoute ensuite 0,5 ml supplémentaire de PBS sur le filtre et les suspensions résultantes sont rassemblées puis centrifugées à 5000 g pendant 5 mn.

Pour empêcher la dégradation de l'ARN, on ajoute 40 unités d'inhibiteur RNase (Boehringer) et on ajoute de l'eau ajustée avec 0,05 % de diethylpyrocarbonate pour inactiver les nucléases jusqu'à un volume final de 50 µl selon le procédé décrit dans Roszak et al. (13) dont le contenu est ici incorporé par référence.

Les cellules sont ensuite lysées par ajout de 50 mg de microbilles de verre (0.5 mm, Biospec Products, Bartlesville, Oklahoma). On secoue les tubes deux fois pendant 20 secondes à 5000 tours par minute, à 10 secondes d'intervalle et on les laisse sur de la glace entre deux mélanges. Après décantation, on transfère 10 µl du lysat dans un nouveau tube.

### c) élimination de l'ADN génomique

On ajoute du-tampon EZ (trousse Perkin Elmer) comme tampon de réaction pour l'enzyme rTth concentré une fois, 2,5 mM de Mn(OAc)2 et, afin d'empêcher l'amplification ultérieure de séquences génomiques contaminantes (14), 10 unités de DNase I ne comportant pas de RNase (Boehringer) jusqu'à atteindre un volume final de 20 µl. Après incubation pendant 10 mn à 37°C, on inactive la DNase en portant la température à 95°C pendant 5 mn.

### d) RT-PCR

Les deux étapes de RT et de PCR sont mises en oeuvre successivement dans le même tube. Pour l'étape de RT, on ajoute, jusqu'à atteindre un volume final de 50 µl, 15 µl du lysat digéré avec la DNase I, le tampon 1 X EZ, 1.4 mM de Mn(OAc)2, 0.3 mM de deoxyribonucléoside triphosphate (Perkin Elmer), 0.4 µM des amorces B1 et B2 (5' CGCTGGAAGGCGACGMRRAG 3'/ 5' CGGAAGTAGAACTGCGGACGGTAG 3')(brins supérieur et inférieur) et 5 unités rTth d'ADN polymérase (Perkin Elmer). Le mélange est mis à incuber pendant 2.5 mn à 95°C, 20-mn à 60°C et 1 mn à 95°C. Pour l'étape PCR, on réalise 40 cycles directement, chaque cycle consistant en 15 secondes à 95°C et 30 secondes à 60°C. On réalise une extension finale pendant 10 mn à 60°C.

### e) détection des faux-positifs

On vérifie s'il y a des faux-positifs dus à la contamination par l'ADN, en ajoutant 5 µl du lysat digéré avec la DNase I, le tampon 1 X Taq (Boehringer), 0.05 mM de déoxyribonucléoside triphosphate, 0.4 µM de chaque amorce B1 et B2 et 2.5U de Taq polymérase (Boehringer) jusqu'à atteindre un volume final de 50 µl.

### f) séparation des produits d'amplification

On sépare 10 µl des produits d'amplification par électrophorèse sur gel horizontal (1 à 2 % d'agarose dans de l'EDTA Tris-acétate) et on visualise par coloration au bromure d'ethidium et illumination aux UV. L'ARN total est aussi analysé comme contrôle RT-PCR positif (il est extrait en utilisant la trousse RNAgentTM (Promega)) et on compare les produits d'amplification obtenus à ceux obtenus à partir de l'ARN total. Une étape facultative peut être rajoutée pour confirmer l'identité des produits amplifiés. Pour cela, on purifie sur gel les bandes de taille attendue ("Trousse Extrait de Gel" (Gel Extract Kit), Qiagen) (fragment de 462 pb), puis on utilise 1 µl de la solution d'ADN comme matrice pour une seconde PCR en utilisant des amorces internes.

### Résultats

On a pu détecter le facteur d'élongation à partir de 10 cellules / ml, ce qui correspond à 5-10 cellules par tube de réaction selon la procédure utilisée (Fig. 1A, bande 5). Aucun transcrit n'est détecté en dessous de ce niveau ainsi que dans le lait non contaminé (Fig. 1A, bandes 6 et 7 respectivement). Après le traitement à la DNase I (Fig. 1A, bande 9), aucun produit de PCR détectable n'est apparu à partir du lysat des cellules. Il semble donc que l'ajout d'une étape de traitement à la DNase I empêche bien l'amplification des séquences génomiques contaminantes.

### EXEMPLE 2 : Mise en oeuvre de la RT-PCR sur un échantillon de lait contaminé à la fois par des cellules de E. coli vivantes et mortes

Quand toutes les cellules sont mortes (cela a été vérifié par mise sur plaque), la détection par RT-PCR est négative (Fig. 2A, bande 1) ce qui confirme que la méthode de RT-PCR permet de détecter seulement les cellules vivantes. Bien que la production de résultats quantitatifs soit difficile par la RT-PCR conventionnelle, une analyse par électrophorèse sur gel a montré une augmentation quantitative de l'intensité des bandes spécifiques lorsque le nombre de cellules viables augmente (Fig. 2A, bandes 2 à 5) ce qui indique une augmentation apparente de l'ADN cible de la procédure RT-PCR au fur et à mesure que le nombre de cellules viables augmente.

### EXEMPLE 3 : Détermination de la présence de cellules vivantes de S. cerevisiae dans du lait (détection d'ARNm codant pour le facteur d'élongation EF-1α).

La procédure de l'exemple 1 est répétée à l'exception des différences suivantes :

Une souche S. cerevisiae S 288C est cultivée à 30°C dans un milieu YPD ("Yeast Peptone Dextrose") contenant 2% de bactopeptone, 1% d'extrait de levure (Laboratoires Difco) et 2% de glucose. Puis on prépare un milieu solide par addition d'agar à 2% (Laboratoires Difco). Une partie des cellules est prélevée pendant la phase exponentielle de croissance. Les cellules sont réparties dans des échantillons de 2ml puis sont exposées à des conditions léthales par incubation à 65°C pendant 15 min.

Lors de la mise en oeuvre de la RT-PCR, les amorces oligonucléotidiques utilisées qui sont spécifiques du facteur d'élongation EF-1α sont les suivantes :

Le même type de résultats est obtenu qu'à l'exemple 1 (Fig. 1B, détection d'un fragment de 657 pb).

### EXEMPLE 4 : Mise en oeuvre de la RT-PCR sur un échantillon de lait contaminé par des cellules de S. cerevisiae à la fois vivantes et mortes.

On observe le même type de résultats qu'à l'exemple 2 (Fig. 2B).

### EXEMPLE 5 : Détermination de la présence de cellules vivantes de M. racemosus dans du lait (détection d'ARNm codant pour le facteur d'élongation EF-1α).

La procédure de l'exemple 1 est répétée sauf qu'à l'étape a) la souche de M. racemosus est cultivée à 30°C dans un milieu YPD contenant 2% de bactepeptone, 1% d'extrait de levure (Laboratoires Difco) et 2% de glucose. Puis on prépare un milieu solide par addition d'agar à 2% (Laboratoires Difco). Une partie des cellules est prélevée pendant la phase exponentielle de croissance. Les cellules sont réparties dans des échantillons de 2ml puis sont exposées à des conditions léthales par incubation à 120°C pendant 30 min.

Des dilutions en série des échantillons sont mises sur plaques sur milieu Agar OGY (MERCK) afin de vérifier la viabilité des cellules et de les compter.

Lors de la mise en oeuvre de la RT-PCR, les amorces nucléotidiques utilisées qui sont spécifiques du facteur d'élongation EF-1α sont les suivantes : Le même type de résultats est obtenu qu'à l'exemple 1 (Fig. 1C, détermination d'un fragment de 498 pb).

### EXEMPLE 6 : Mise en oeuvre de la RT-PCR sur un échantillon de lait contaminé à la fois par des cellules de M. racemosus vivantes et mortes.

On observe le même type de résultats qu'à l'exemple 2 (Fig. 2C).

### EXEMPLE 7 : Détermination de la présence simultanée de cellules vivantes de E. coli, S. cerevisiae et M. racemosus.

Les gènes EF-Tu de E.coli et EF-1α de S.cerevisiae et M.racemosus ont été détectés simultanément dans une seule réaction. On a détecté simultanément par RT-PCR des produits d'amplification de taille attendue (respectivement fragments de 462 pb (E. coli) 657 pb (S. cerevisiae) et 498-pb (M. racemosus)), en utilisant du lait contaminé à la fois par E.coli, S.cerevisiae et M.racemosus. Pour cela, on a placé deux (Fig.3 bandes 4, 5 et 6) ou trois (Fig. 3 bande 7) paires d'amorces dans le même mélange réactionnel sans modification des conditions de la RT-PCR.

Ainsi, il apparaît que la RT-PCR est une méthode particulièrement puissante pour la détection de contaminants viables en raison de sa forte capacité à augmenter les sensibilités de détection et sa rapidité de mise en oeuvre. Un niveau de détection de 10 cellules / ml de lait a pu être atteint et l'ensemble du procédé de RT-PCR, y compris lavage et filtration de l'échantillon de lait, a pu être réalisé en 4 heures sans nécessiter d'étape préliminaire d'enrichissement. Le choix du gène cible est important. La sélection d'amorces spécifiques de la région codante du gène du facteur d'élongation s'est ainsi révélée particulièrement avantageuse.

### BIBLIOGRAPHIE

1. BLACKBURN, C. de W. 1993. A review : rapid and alternative methods for the detection of salmonellas in food. J. Appl. Bacteriol. 75:199-214.
2. WEIJLAND, A., K. HARMARK, R.H. COOL, P.H. ANBAGH, et A. PARMEGGIANI. 1992. Elongation factor Tu: a molecular switch in protein synthesis. Mol. Microbiol. 6:683-688.
3. MERRICK, W.C. 1992. Mechanism and regulation of eukaryotic protein synthesis. Microbiol. Rev. 56:291-315.
4. THOMPSON, R.C. 1988. EF-Tu provides an internal kinetic standard for translational accuracy. Trends Biochem. Sci. 13:91-93.
5. BOSH, L., B. KRAAL, P.H. VAN DER MEIDE, F.J. DUISTERVINKEL, et J.M. VAN NOORT. 1983. The elongation factor EF-Tu and its two encoding genes. Prog. Nucleic Acid Res. Mol. Biol. 30:91-126.
6. COTRELLE, P., M. COOL, P. THURIADX, V.L. PRICE, D. THIELE, J.M. BUHLER, et P. FROMAGEOT. 1985. Either one of the two yeast EF-1α genes is required for cell viability. Current Genetics. 9:693-697.
7. GORDON, J. 1970. Regulation of the in vivo synthesis of the polypeptide chain elongation factors in Escherichia coli. Biochemistry 9:912-917.
8. SCHIRMAIER, F., et P. PHILIPPSEN. 1984. Identification of two genes coding for the translation elongation factor EF-1α of S. cerevisiae. EMBO J. 3:3311-3315.
9. LUDWIG, W., M. WEIZENEGGER, D. BETZL, E. LEIDL, T. LENZ, A. LUDVIGSEN, D. MOLLENHOFF, P. WENZIG et K.H. SCHLEIFER. 1990. Complete nucleotide sequences of seven eubacterial genes coding for the elongation factor Tu: functional, structural and phylogenetic evaluations. Arch. Microbiol. 153:241-247.
10. CATTANEO, R. 1991. Different types of messenger RNA editing. Annu. Rev. Genet. 25:71-88.
11. NEIDHARDT, F.C., J.L. INGRAHAM, et M. SCHAECHTER. 1990. Physiology of bacterial cell : a molecular approach Sinauer Associates Inc., Sunderland, Mass.
12. COORAY, K. J., T. NISHIBORI, H. XIONG, T. MATSUYAMA, M. FUJITA, et M.-MITSUYAMA. 1994. Detection of multiple virulence-associated genes of Listeria monocytogenes by PCR in articifially contaminated milk samples. Appl. Environ. Microbiol. 60:3023-3026.
13. ROSZACK, D.B. et R.R. COLWELL. 1987. Survival strategies of bacteria in natural environment. Microbiol. Rev. 51:365-379.
14. TONG, J., S. BENDAHHAR, H. CHEN, et W.S. AGNEW. 1994. A simplified method for single RT-PCR that can detect and distinguish genomic DNA and mRNA transcripts. Nucl. Acid. Res. 22:3253-3254.

## Revendications

1. Procédé de mise en évidence de contaminants microbiologiques vivants dans un échantillon de produit à usage alimentaire selon lequel on détecte dans ledit échantillon la présence d'ARNm codant pour la synthèse d'une protéine intervenant dans la synthèse des protéines par lesdits contaminants.

2. Procédé selon la revendication 1 pour la mise en évidence de façon non spécifique de contaminants vivants appartenant à des espèces différentes d'une famille de microorganismes, **caractérisé en ce que** ledit ARNm code pour la synthèse d'une protéine dont la structure primaire est au moins partiellement conservée entre lesdites espèces différentes.

3. Procédé selon la revendication 1 pour la mise en évidence de façon spécifique de contaminants vivants appartenant à des espèces différentes d'une famille de microorganismes, **caractérisé en ce qu'**on détecte la présence de différents ARNm codant respectivement pour la synthèse d'une protéine dont la structure primaire n'est pas conservée entre lesdites espèces différentes.

4. Procédé selon l'une des revendications 1 à 3, **caractérisé en ce que** ledit ARNm code pour un facteur d'élongation.

5. Procédé selon l'une des revendications 1 à 4, **caractérisé en ce qu'**on détecte la présence d'ARNm par mise en oeuvre de la technique RT-PCR.

6. Procédé selon la revendication 5, **caractérisé en ce que** les amorces oligonucléotidiques utilisées dans la PCR sont spécifiques de la région codante d'un gène codant pour un facteur d'élongation.

7. Procédé selon la revendication 6 pour la mise en évidence des bactéries, **caractérisé en ce que** ledit facteur d'élongation est le facteur EF-Tu.

8. Procédé selon la revendication 7, **caractérisé en ce qu'**on utilies la paire d'amorces oligonucléotidiques suivante :

9. Procédé selon la revendication 6 pour la mise en évidence des levures ou des champignons, **caractérisé en ce que** ledit facteur d'élongation est le facteur EF-1α.

10. Procédé selon la revendication 9 pour la mise en évidence des levures, **caractérisé en ce qu'**on utilise la paire d'amorces oligonucléotidiques suivante :

11. Procédé selon la revendication 9 pour la mise en évidence de champignons, **caractérisé en ce qu'**on utilise la paire d'amorces oligonucléotidiques suivante :

12. Procédé selon l'une quelconque des revendications précédentes, **caractérisé en ce que** :
a) on prélève un échantillon du produit à usage alimentaire à tester;
b) on lyse les cellules ;
c) on effectue une transcription inverse ;
d) on effectue des cycles d'amplification à l'aide d'amorces oligonucléotidiques spécifiques de la région codante d'un gène codant pour un facteur d'élongation ;
e) on sépare les produits d'amplification ;
f) on visualise les produits d'amplification ;
g) on compare les produits tels que visualisés à l'étape f) avec les produits d'amplification obtenus à partir d'ARNm pur.

13. Procédé selon la revendication 12, **caractérisé en ce qu'**après l'étape b) on ajoute une étape b') visant à éliminer l'ADN présent dans l'échantillon.

14. Procédé selon la revendication 13, **caractérisé en ce qu'**il comporte une étape visant à vérifier l'absence de faux positifs liés à la présence d'ADN.

15. Procédé selon l'une des revendications 5 à 14 précédentes pour la détection spécifique de bactéries et/ou de levures et/ou de champignons, **caractérisé en ce qu'**on utilise au moins deux des paires d'amorces B1/B2, L1/L2, M1/M2, les conditions de la RT-PCR restant inchangées.

## Patentansprüche

1. Verfahren zum Nachweis von lebenden mikrobiologischen Verunreinigungen in einer Probe eines Produkts zur Verwendung als Nahrungsmittel, gemäß welchem man in der Probe die Anwesenheit von mRNS nachweist, welche für die Synthese eines Proteins kodiert, das an der Synthese von Proteinen durch die Verunreinigungen teilnimmt.

2. Verfahren nach Anspruch 1 für den Nachweis von lebenden Verunreinigungen, die verschiedenen Spezies einer Familie von Mikroorganismen angehören, auf nicht spezifische Weise, **dadurch gekennzeichnet, dass** die mRNS für die Synthese eines Proteins kodiert, dessen Primärstruktur mindestens teilweise zwischen den verschiedenen Spezies konserviert ist.

3. Verfahren nach Anspruch 1 für den Nachweis von lebenden Verunreinigungen, die verschiedenen Spezies einer Familie von Mikroorganismen angehören, auf spezifische Weise, **dadurch gekennzeichnet, dass** man die Anwesenheit verschiedener mRNS nachweist, die jeweils für die Synthese eines Proteins kodieren, dessen Primärstruktur unter den verschiedenen Spezies nicht konserviert ist.

4. Verfahren nach einem der Ansprüche 1 bis 3, **dadurch gekennzeichnet, dass** die mRNS für einen Verlängerungsfaktor kodiert.

5. Verfahren nach einem der Ansprüche 1 bis 4, **dadurch gekennzeichnet, dass** man die Anwesenheit der mRNS mittels Durchführung der RT-PCR-Technik nachweist.

6. Verfahren nach Anspruch 5, **dadurch gekennzeichnet, dass** die Oligonukleotid-Primer, die in der PCR verwendet werden, spezifisch für die kodierende Region eines Gens sind, welches für einen Verlängerungsfaktor kodiert.

7. Verfahren nach Anspruch 6 für den Nachweis von Bakterien, **dadurch gekennzeichnet, dass** der Verlängerungsfaktor der Faktor EF-Tu ist.

8. Verfahren nach Anspruch 7, **dadurch gekennzeichnet, dass** man das folgende Oligonukleotid-Primerpaar verwendet:

9. Verfahren nach Anspruch 6 für den Nachweis von Hefen oder Pilzen, **dadurch gekennzeichnet, dass** der Verlängerungsfaktor der Faktor EF-1α ist.

10. Verfahren nach Anspruch 9 für den Nachweis von Hefen, **dadurch gekennzeichnet, dass** man das folgende Oligonukleotid-Primerpaar verwendet:

11. Verfahren nach Anspruch 9 für den Nachweis von Pilzen, **dadurch gekennzeichnet, dass** man das folgende Oligonukleotid-Primerpaar verwendet:

12. Verfahren nach irgendeinem der vorangehenden Ansprüche, **dadurch gekennzeichnet, dass**:
a) man eine Probe des Produkts zur Verwendung als Lebensmittel zum Testen entnimmt;
b) man die Zellen lysiert;
c) man eine inverse Transkription bewirkt;
d) man Amplifikationszyklen mit Hilfe von spezifischen Oligonukleotid-Primern der kodierenden Region eines Gens, das für einen Verlängerungsfaktor kodiert, bewirkt;
e) man die Amplifikationsprodukte abtrennt;
f) man die Amplifikationsprodukte sichtbar macht;
g) man die so im Schritt f) sichtbar gemachten Produkte mit den Amplifikationsprodukten vergleicht, die ausgehend von reiner mRNS erhalten werden.

13. Verfahren nach Anspruch 12, **dadurch gekennzeichnet, dass** man nach dem Schritt b) einen Schritt b') hinzufügt, der auf die Eliminierung von in der Probe vorliegender DNS gerichtet ist.

14. Verfahren nach Anspruch 13, **dadurch gekennzeichnet, dass** es einen Schritt umfasst, der darauf gerichtet ist, die Abwesenheit von falsch Positiven, die mit der Anwesenheit von DNS verbunden sind, zu verifizieren.

15. Verfahren nach einem der vorangehenden Ansprüche 5 bis 14 für den spezifischen Nachweis von Bakterien und/oder Hefen und/oder Pilzen, **dadurch gekennzeichnet, dass** man mindestens zwei der Primerpaare B1/B2, L1/L2, M1/M2 verwendet, wobei die Bedingungen der RT-PCR unverändert bleiben.

## Claims

1. Process for detecting live microbiological contaminants in a food product sample, according to which the presence of mRNA coding for the synthesis of a protein involved in the synthesis of proteins by the said contaminants is detected in the said sample.

2. Process according to Claim 1 for non-specifically detecting live contaminants belonging to different species of a family of microorganisms, **characterized in that** the said mRNA codes for the synthesis of a protein whose primary structure is at least partially conserved between the said different species.

3. Process according to Claim 1 for specifically detecting live contaminants belonging to different species of a family of microorganisms, **characterized in that** the presence is detected of different mRNAs, each of which codes for the synthesis of a protein whose primary structure is not conserved between the said different species.

4. Process according to one of Claims 1 to 3, **characterized in that** the said mRNA encodes an elongation factor.

5. Process according to one of Claims 1 to 4, **characterized in that** the presence of mRNA is detected by performing the RT-PCR technique.

6. Process according to Claim 5, **characterized in that** the oligonucleotide primers employed in the PCR are specific for the coding region of a gene which encodes an elongation factor.

7. Process according to Claim 6 for detecting bacteria, **characterized in that** the said elongation factor is the EF-Tu factor.

8. Process according to Claim 7, **characterized in that** the following pair of oligonucleotide primers is employed:

9. Process according to Claim 6 for detecting yeasts or fungi, **characterized in that** the said elongation factor is the factor EF-1α.

10. Process according to Claim 9 for detecting yeasts, **characterized in that** the following pair of oligonucleotide primers is employed:

11. Process according to Claim 9 for detecting fungi, **characterized in that** the following pair of oligonucleotide primers is employed:

12. Process according to any one of the preceding Claims, **characterized in that**:
a) a sample of the food product to be tested is withdrawn;
b) the cells are lysed;
c) a reverse transcription is carried out;
d) amplification cycles are carried out using oligonucleotide primers which are specific for the coding region of a gene which encodes an elongation factor;
e) the amplification products are separated;
f) the amplification products are visualized;
g) the products as visualized in step f) are compared with the amplification products which are obtained from pure mRNA.

13. Process according to Claim 12, **characterized in that** a step b'), which is directed toward removing the DNA which is present in the sample, is added after step b).

14. Process according to Claim 13, **characterized in that** it includes a step which is directed toward verifying the absence of false positives linked to the presence of DNA.

15. Process according to one of the preceding Claims 5 to 14 for specifically detecting bacteria and/or yeasts and/or fungi, **characterized in that** at least two of the primer pairs B1/B2, L1/L2 and M1/M2 are employed, with the RT-PCR conditions remaining unchanged.
